# EUROPEAN PATENT APPLICATION

(11) **EP 3 678 144 A1**
(43) Date of publication of application: **08.07.2020**
(21) Application number: 19173152.0
(22) Date of filing: 12.02.2010
(51) Int. Cl.: G16H 30/20, G16H 20/40

(54) **METHOD AND APPARATUS FOR MANUFACTURING AN IMPLANT**

(30) Priority: 13.02.2009 US 371096; 12.06.2009 US 483807
(62) Divisional of application: 10705951.1
(71) Applicant: Biomet Manufacturing, LLC, Warsaw, IN 46582 (US)
(72) Inventor: BELCHER, Nathan, E., Olivette, MO Missouri 63132 (US); METZGER, Robert, Wakarusa, IN Indiana 46573 (US); CATANZARITE, Joshua, B., Wapsaw, IN Indiana 46582-6964 (US)
(74) Representative: Mays, Julie

(57) **Abstract**

An orthopedic implant manufacturing method. The method includes preparing a pre-operative surgical plan for a specific patient, the surgical plan including a three-dimensional image of a patient's joint indicating at least one resection plane, communicating the surgical plan to a surgeon of the patient, and receiving approval of the surgical plan and the resection plane by the surgeon. The method also includes providing automated osteophyte/protrusion removal control for surgeon manipulation, receiving a modified three-dimensional image of a patient's joint indicating an osteophyte/protrusion removal and a recommendation for a corresponding selected orthopedic implant from the surgeon, and requesting manufacture of the selected orthopedic implant.

## Description

### CROSS-REFERENCE TO RELATED APPLICATIONS

This application claims priority to U.S. Application No. 12/483,807, filed June 12, 2009, which is a continuation-in-part of U.S. Application No. 12/103824, filed April 16, 2008, which claims the benefit of U.S. Provisional Application No. 60/912178, filed April 17, 2007. Additionally, this application claims priority to U.S. Application No. 12/371,096, filed February 13, 2009, which is a continuation-in-part of U.S. Application No. 12/103,824, filed April 16, 2008, which claims the benefit of U.S. Provisional Application No. 60/912,178, filed April 17, 2007. All of the disclosures of the above applications are incorporated herein by reference.

### INTRODUCTION

The present teachings provide a mobile-interactive apparatus and method to receive input from a user regarding a selected patient procedure.

### SUMMARY

The present teachings provide an orthopedic implant manufacturing method. The method includes preparing a preliminary pre-operative surgical plan for a specific patient, communicating the plan to a surgeon of the patient, and receiving an orthopedic implant design recommendation of the surgeon. The implant design recommendation can include selecting one of first, second or third options. The first option being a patient-specific implant, the second option being a semi-custom implant, and the third option being an off-the-shelf or standard production implant. The method further includes sending a request for manufacturing the selected implant to a manufacturing center, receiving the implant, and forwarding the implant for implantation.

In another aspect, the orthopedic implant manufacturing method includes providing a generic casting of a specific implant component, the generic casting having at least one geometric feature that can be machined to a plurality of different sizes of the implant component, the generic casting including size-independent features of the specific component, and machining the component to a patient-specified size.

The present teachings also provide a device that includes a generic casting for a specific implant component, the generic casting being intermediate between stock material and a specific size implant component. The generic casting includes at least one size-independent feature of the implant component, and at least one feature machinable to size/shape for a specific patient.

The present teachings also provide an orthopedic implant manufacturing method. The method includes preparing a pre-operative surgical plan for a specific patient. The surgical plan including a three-dimensional image of a patient's joint indicating at least one resection plane, communicating the surgical plan to a surgeon of the patient, and receiving approval of the surgical plan and the resection plane by the surgeon. The method also includes providing automated osteophyte/protrusion removal control for surgeon manipulation, receiving a modified three-dimensional image of a patient's joint indicating an osteophyte/protrusion removal and a recommendation for a corresponding selected orthopedic implant from the surgeon, and requesting manufacture of the selected orthopedic implant.

In another aspect, the method includes preparing a pre-operative surgical plan for a specific patient, the surgical plan including a three-dimensional image of a patient's joint indicating at least one resection plane, communicating the surgical plan to a surgeon of the patient, receiving approval of the surgical plan and the resection plane by the surgeon, and identifying a location of at least one osteophyte/protrusion on the three-dimensional image of a patient's joint. The method also includes providing a plurality of depth contours in relation to the osteophyte/protrusion, providing at least one graphical removal tool associated with the osteophyte/protrusion for manipulation by the surgeon, receiving a modified three-dimensional image of a patient's joint indicating an osteophyte/protrusion removal and a recommendation for a corresponding selected orthopedic implant from the surgeon, and requesting manufacture of the selected orthopedic implant.

In a further aspect, the method includes preparing a pre-operative surgical plan for a specific patient, the surgical plan including a three-dimensional image of a patient's joint indicating at least one resection plane, identifying a location of at least one osteophyte/protrusion on the three-dimensional image of a patient's joint, providing a plurality of depth contours in relation to the osteophyte/protrusion, and providing at least one graphical removal tool associated with the osteophyte/protrusion for manipulation by a user. The method also includes, communicating the surgical plan to a user, receiving a modified three-dimensional image of a patient's joint indicating an osteophyte/protrusion removal and a recommendation for a corresponding selected orthopedic implant from a user, and requesting manufacture of the selected orthopedic implant.

Further areas of applicability of the present teachings will become apparent from the description provided hereinafter. It should be understood that the description and specific examples are intended for purposes of illustration only and are not intended to limit the scope of the present teachings.

### BRIEF DESCRIPTION OF THE DRAWINGS

The present teachings will become more fully understood from the detailed description and the accompanying drawings, wherein:
FIG. 1 is a flowchart of an implant manufacturing method according to the present teachings;
FIG. 2 is a diagram illustrating a computer interface for an implant manufacturing method according to the present teachings;
FIG. 3 is perspective view of a generic casting of an implant according to the present teachings;
FIG. 4 is a side view of a generic casting according to the present teachings;
FIG. 5 is a plan view of a generic casting according to the present teachings;
FIG. 6 is a flow chart for an osteophyte/protrusion removal control method according to the present teachings;
FIG. 7 is a representative image of a patent's anatomy showing osteophyte/protrusion control tools for modifying the image;
FIGS. 8 and 9 are representative images of a patent's anatomy showing exemplary osteophyte/protrusion locations;
FIG. 10 is a representative image of a patent's anatomy showing representative depth control selections for surgeon manipulation;
FIGS. 11 and 12 are representative images of a patent's anatomy after osteophyte/protrusion removal with exemplary implants attached thereon;
Figs. 13A and 13B illustrate a flowchart of a method of implant and guide design;
Fig. 14 is a schematic view of hardware and a user; and
Fig. 15 is a plan view of a display of a device.

### DESCRIPTION OF VARIOUS ASPECTS

The following description is merely exemplary in nature and is in no way intended to limit the present teachings, applications, or uses. For example, although some of the present teachings are illustrated for a knee implant, the present teachings can be used for any orthopedic implant.

The present teachings provide a manufacturing method that integrates patient's anatomic and medical information with interactive participation by a surgeon to select and manufacture an implant and, optionally, related surgical instruments, for a particular patient from generally three options: a custom made implant specific to the patient, an implant that is only partially custom-made or a semi-custom implant, and a standard off-the shelf implant. Similarly, off-the-shelf, custom-made, or semi-custom-made instrumentation (e.g. alignment guides, drill guides, cutting guides or other instruments) can be selected and manufactured, as recommended by the surgeon, for the surgical procedure. All the implant components, alignment guides, and other disposable instruments can be included in a package provided to a surgeon for a specific patient.

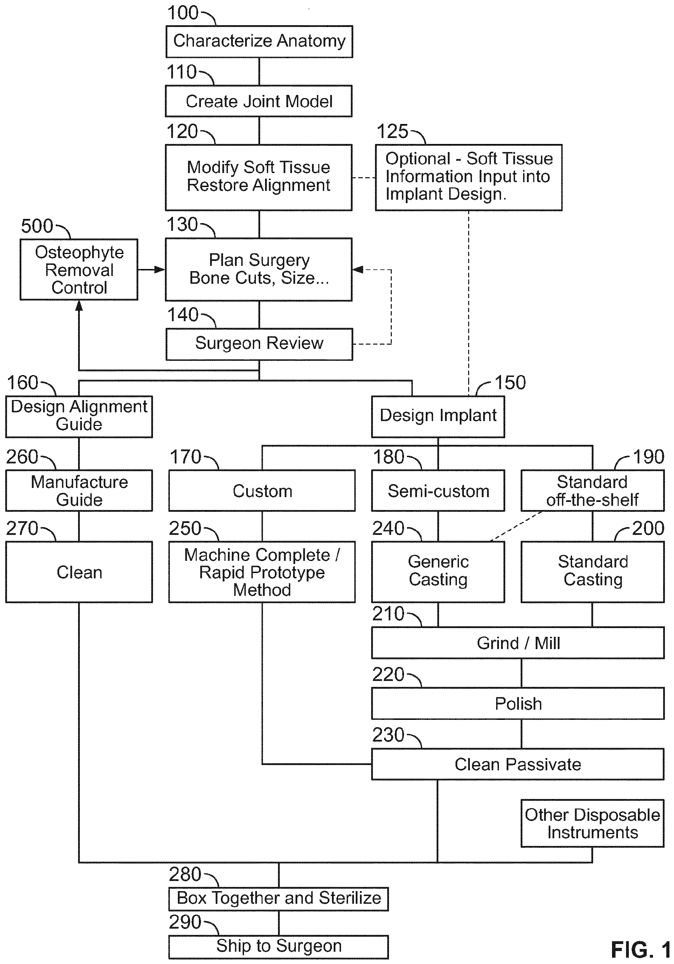

Referring to FIG. 1, an exemplary flowchart of an interactive implant manufacturing method according to the present teachings is illustrated. The portion of the patient's anatomy related to the orthopedic procedure and the implant is characterized and detailed at 100. The characterization can be performed with various imaging methods capable of obtaining a representation of the affected anatomy, including, for example, soft and hard tissues. The tissues can include bone, bone joints with or without cartilage, ligaments, or other soft tissue. The imaging methods can include, for example, MRI, CT, ultrasound, radiography or X-ray, cameras and other devices.

The image information for the patient can be obtained at a medical facility or a doctor's office and can be sent to the manufacturer in an electronic and/or digital form contained. The image information can be stored on a physical medium, such as a CD, DVD, flash memory device (e.g. memory stick, compactflash, secure digital card), or other storage device. The information may alternatively, or in addition, be transmitted electronically with the Internet or worldwide web using appropriate transfer protocols. Also, electronic transmissions can include e-mail or other digital transmission to any appropriate type of computer device, smart phone, PDA or other devices in which electronic information can be transmitted.

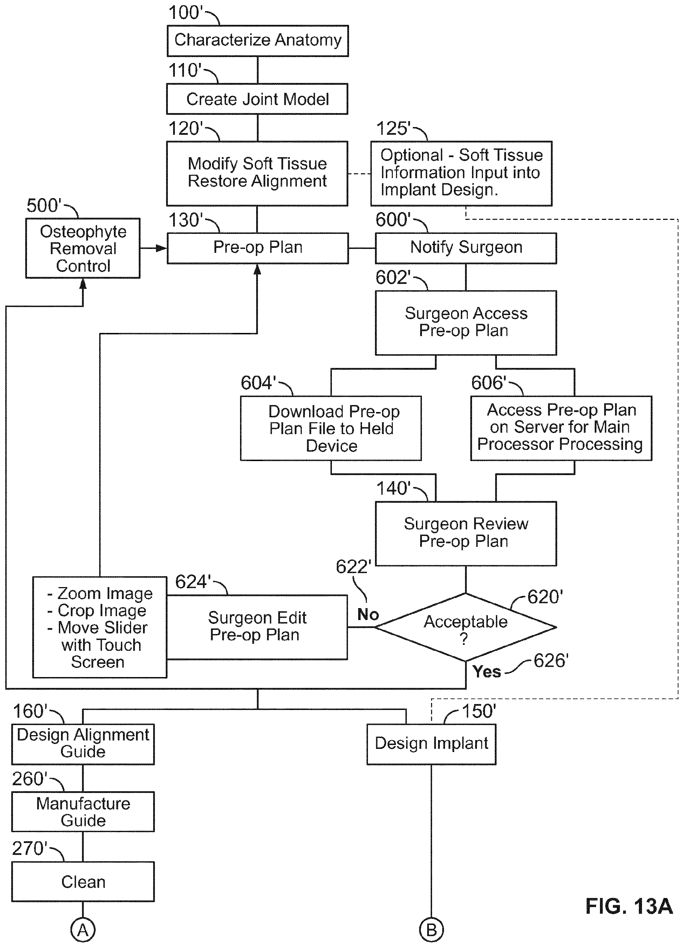

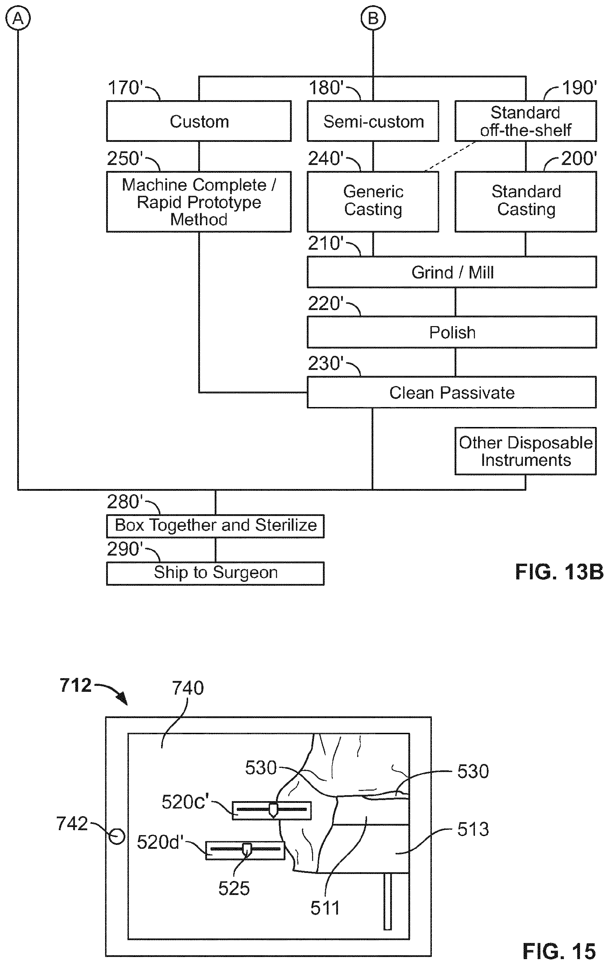

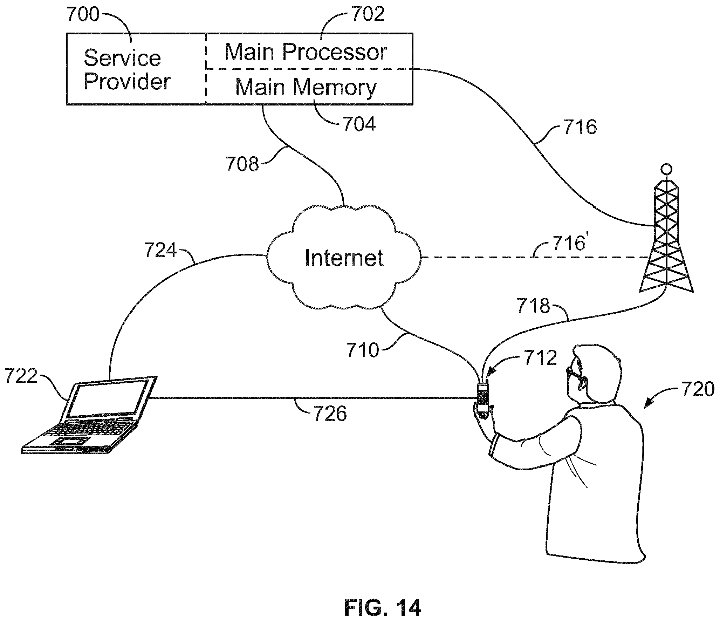

Appropriate handheld devices (used as illustrated in Fig. 13), can include handheld mobile device or portable communication devices, such as the iPhone® handheld mobile device sold by Apple Inc., a corporation of California, USA; the LG Shine® handheld mobile device sold by LG Corp. a corporation of REPUBLIC OF KOREA; or the Blackberry Bold® handheld mobile device sold by Research In Motion Limited a corporation of CANADA. The handheld device can be those that are held in the palm of a hand of a user, such as a surgeon (see Fig 14). The surgeon can then enter data with a stylus, keyboard, touch screen, etc. The handheld device can use local area networks, cell phone networks, or other data transmission systems to communicate with a main memory and processor of a service provider (see Fig. 14).

Appropriate handheld devices can provide access to electronic communication or file transfer protocols, such as internet or electronic mail, to transfer or access information files. The handheld devices can have installed programs that can be used to manipulate the information files, as discussed herein. Alternatively, or in addition thereto, the handheld devices can access servers that process data files while receiving input through the handheld devices and displaying images to the surgeon or user via the handheld device. In certain instances, the handheld device may only be a client that does not process and edit a data file of the pre-op plan.

With continued reference to FIG. 1, at 110, the information collected at 100 can be used to create a three-dimensional model or image of the bone or joint with or without associated soft tissue or related anatomy using commercially available computer modeling software from various vendors or developers, such as, for example, from Materialise USA, Ann Arbor, Michigan. The three-dimensional model of the patient's anatomy can be viewed on a computer display or other electronic screen and can also reproduced as a hard copy on film or other medium and viewed by direct or indirect or backlight illumination. The model can be sized for viewing on any appropriate screen size and may be cropped, rotated, etc. as selected by the individual (e.g. the surgeon) viewing the screen.

At 120, soft tissue associated with the affected anatomy can be modified, or removed or repaired, to restore alignment of the joint, for example, or to remove torn or diseased tissue, or to cut or repair ligaments, or to provide natural or artificial ligament grafts. Soft tissue information can be optionally used as an additional design parameter or input for the implant design, at 125. For example, a custom or patient-specific bearing articulation of a knee joint can be designed based on the kinematic profile and the soft tissue/ligament information available for a particular patient. Further, kinematic information for the patient can be obtained by an actual gait analysis of the patient, and can also be obtained by computer modeling software that uses the MRI images of the patient's joints and associated ligaments, muscle or other soft tissue to derive kinematic analysis of the patient and corresponding recommendations for soft tissue modification, such as releasing a ligament, for example. Such software is commercially available from the Biomechanics Research Group, Inc., of San Clemente, CA.

At 130, a preliminary pre-operative plan of the surgical procedure can be prepared for surgeon or other medical user or technician review, including the planning of various bone resections, sizes and types of implants, and various geometric requirements including relevant dimensions, such as height, width, orientation of particular features, etc. The preliminary pre-operative surgical plan can include a recommendation of particular implants and associated instruments to be used in the surgical procedure, as discussed below. The preliminary pre-operative surgical plan can be in the form of digital images that can be viewed interactively using a computer modeling software, such as the software referenced above. The preliminary pre-operative plan and any further changes or a finalized pre-operative plan can be a plan devised to obtain a healthy or as close to healthy anatomical orientation after an operative procedure. The healthy anatomy can be based on natural or pre-injury anatomy or mechanically correct or efficient anatomical orientation.

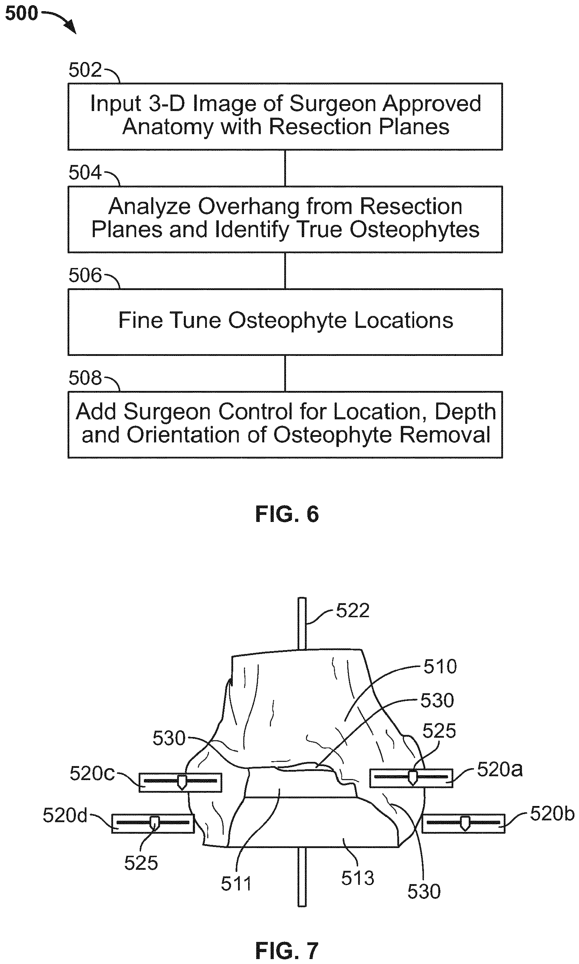

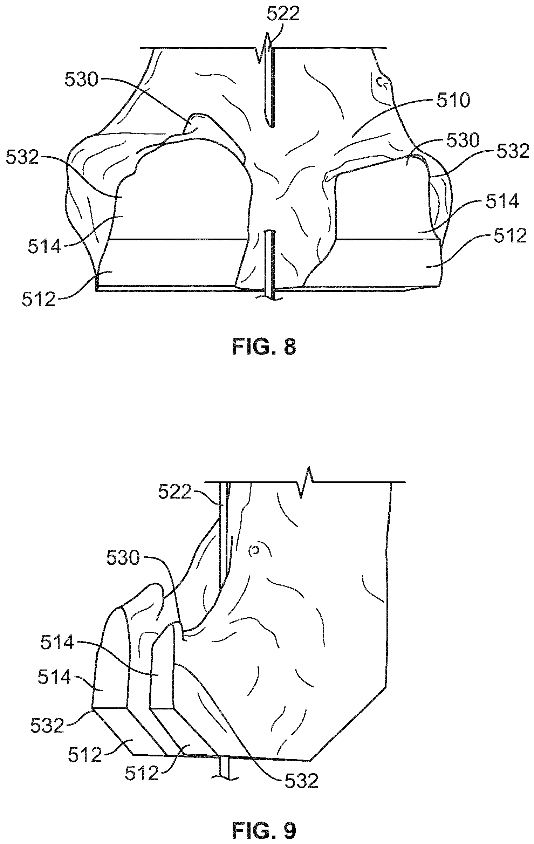

At 140, the preliminary pre-operative surgical plan can be submitted to the surgeon (or other user) for review, either electronically or by land mail, and either in digital or hard copy form, as discussed above in connection with transmitting imaging information. In particular, the surgeon can review the resection planes shown in image of the patient's anatomy, make changes in the location, size and orientation of the resection planes and, generally, work interactively until the pre-operative plan from 130 is surgeon-approved. Specifically, the surgeon may approve the image of the patient's anatomy showing corresponding resection planes. As shown in FIGS. 7 and 8, the patient's anatomy 510 can be, for example, a distal femur with approved resection planes including medial and lateral anterior chamfer planes 513, medial and lateral anterior cut planes 511, medial and lateral posterior chamfer planes 512 and medial and lateral posterior cut planes 514. Following the surgeon's approval of the anatomy and the resection planes at 140, the surgeon is provided with the opportunity to remove one or more osteophytes/protrusions from the image of the patient's anatomy 510 at surgeon-selected locations and depths at 500 (See FIG. 6). Removal of such protrusions and smoothening of the joint surface that receives the implant can parallel the intra-operative joint preparation by the surgeon and improve the actual fit of a surgeon-selected implant, whether patient-specific, semi custom, or off the shelf.

An automated osteophyte/protrusion removal control module 500 can be incorporated in the planning stage of the manufacturing method illustrated in FIG. 1. The automated osteophyte/protrusion removal control module 500 can be provided as a separate pre-operative planning module, as shown in FIG. 6, or it can be incorporated and/or fully integrated with the manufacturing method illustrated in FIG. 1.

Certain parts of the bone, including various bone bumps, protrusions, growths and osteophytes can be generally removed from the three-dimensional reconstruction of a patient's anatomy before designing a patient-specific implant or semi-custom implant, or before selecting an off the shelf implant. The automated osteophyte/protrusion removal control module can replace a time-consuming and potentially less accurate manual modification of the three-dimensional image to remove such bone growths or osteophytes by an experienced image or CAD technician. The automated osteophyte/protrusion removal control module 500 can provide more accurate and faster removal of such bone irregularities, which can vary in shape, location and size from patient to patient. It will be appreciated that the osteophyte/protrusion removal control module 500 can be used for smoothing out a bone surface by removing any type of bone protrusion, including bumps, irregularities and osteophytes. According to the present teachings, osteophytes are illustrated as exemplary, but not exclusive, candidates for complete or partial removal.

Referring to FIG. 6, the osteophyte/protrusion removal control module 500 can start 502 with an input of the three-dimensional image of the patient's anatomy 510 including resection planes, as shown in FIGS. 7-9, after review and approval of the resection planes by the surgeon (or other user, including other professionals or technicians) at 140 of FIG. 1. In the exemplary illustration of FIG. 7, the image of the patient's anatomy 510 can be analyzed to identify osteophyte/protrusion locations 530 (at 504 of FIG. 6) by determining tissue or bone overhang protruding past outer edges 532 of the various resection planes, such as the resection planes illustrated at 511, 513, 512 and 516 in FIGS. 7-9. If such osteophyte/protrusions 530 extend beyond the edges of the resection planes in the direction of the planned or anticipated implant location, the osteophyte/protrusions 530 can interfere with implant fitting.

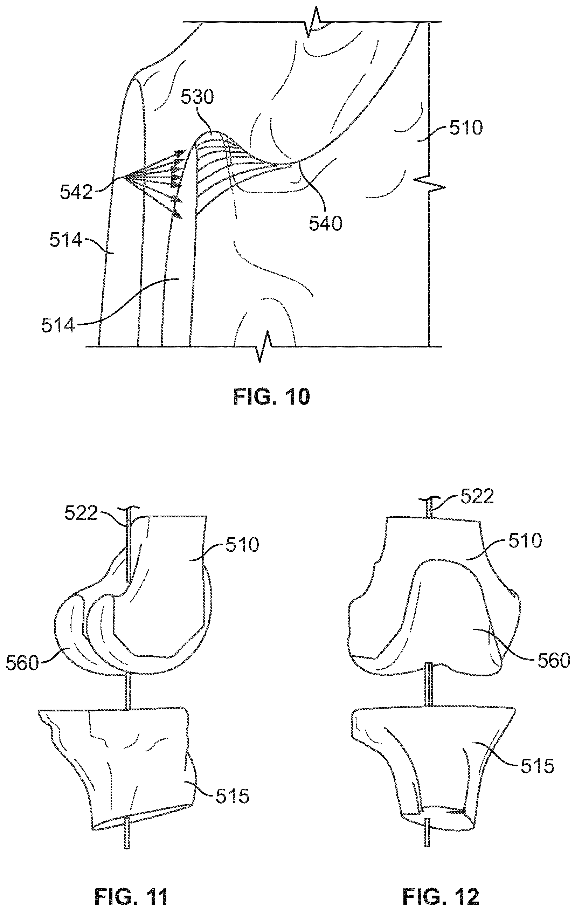

Referring to FIGS. 6, 7 and 10, in addition to identifying the location of osteophytes/protrusions 530, the osteophyte/protrusion removal control module 500 can provide visual control for the surgeon to select the aggressiveness of osteophyte/protrusion removal, or the degree of smoothening and/or flattening of the corresponding joint anatomy. Specifically, by fine-tuning the osteophyte/protrusion locations, at 506 of FIG. 6, the surgeon can control the depth of the osteophyte/protrusion removal in a continuous or discrete manner. In one aspect, a landmark location 540 for each osteophyte/protrusion 530 can be identified and pegged for measuring from and initiating a continuous series of constant or variable depth contours 542 to aid the surgeon in selecting the depth of osteophyte/protrusion removal. The depth contours can be automatically generated by the computer software that generates a three-dimensional model or image of the anatomy, such as the software commercially available, for example, from Materialise USA, Ann Arbor, Michigan. The landmark location 540 can be a location of lowest possible depth in the vicinity of the identified osteophyte/protrusion, a minimum, or a valley location, as shown in FIG. 10. Although the depth contours 542 are shown as discrete in FIG. 10, it will be appreciated that a continuous removal control can be provided, such that the surgeon can exercise unlimited choices of depth contours for removal. The depth contours 542 can represent curved smoothed-out surfaces under the original osteophyte/protrusion 530 and can be exposed after an overlying area is shaved or peeled in the image 510 by the operation of graphical or visual removal tools provided on the image 510. The surgeon or other user can manipulate the graphical removal tools with a user interface, such as a mouse, touch screen, joystick, slide pad, or other user interface.

Referring to FIG. 7, various visual removal tools can be provided for on-screen manipulation and control by the surgeon, at 508 of FIG. 6. For example, a removal tool corresponding to each edge of a resection plane can be provided and used to visually/graphically remove a portion of an osteophyte/protrusion associated with a particular edge 532. In FIG. 7, four such exemplary removal tools 520a, 520b, 520c, 520d (collectively referenced as 520) are shown, each removal tool associated with an edge of a resection plane, such as lateral and medial chamfer plane and lateral and medial cut plane. Although the removal tools 520 are illustrated as straight sliders in FIG. 7, the amount removed follows a depth contour 542, as illustrated in FIG. 10. The removal tools 520 can include a visual indicator 525 that can provide information to the surgeon in the form of a number on a scale indicative of the depth of aggressiveness of osteophyte/protrusion removal. In another aspect, the indicator 525 can provide visual information in terms of variable color in shades gradually changing from minimum depth removal (green, for example) to maximum depth removal (red, for example).

After the surgeon completes the osteophyte/protrusion removal, the surgeon can manipulate and superimpose implant images in relation to the modified patient's anatomy 510. In FIGS. 11 and 12, exemplary images of a resected femur 510 and tibia 515 referenced in relation to a mechanical axis 522 are illustrated. The femur image illustrates the patient's anatomy 510 after the osteophytes/protrusions 530 shown in FIGS. 8 and 9 have been removed and a femoral component 560 is placed on the resulting smoothed out surface that follows one of the depth contours 542 shown in FIG. 9.

Based on the preliminary pre-operative surgical plan and the patient information, the surgeon can make a recommendation regarding the design of the implant at 150, and any desired associated alignment guides at 160. At 150, the surgeon can recommend a method of designing an implant. Specifically, the surgeon can select one of the following three options: a first option of a custom or patient-specific implant at 170 or a second option of a semi-custom made implant at 180, or a third option of a standard or off-the-shelf implant at 190. It will be appreciated that, based on the surgeon's recommendation at 140, the preliminary pre-operative surgical plan can be modified at 130 and then resubmitted to the surgeon for approval.

A custom-made implant is a patient-specific, one of a kind implant specifically made for a particular patient, and consequently there is no inventory associated with such implant. Standard or off-the-shelf-implants are available and stocked in a number of sizes, typically six or more, and a number of configurations or types, including bilateral or unilateral implants, constrained, semi-constrained, mobile, etc. Because of the variety of sizes and configurations that are kept in stock to be accommodate different patients, a large inventory of standard implants is created, and several molds for each type and size of implant may be used. As described below in detail, semi-custom implants provide an intermediate solution between custom-made and off-the-shelf implants. Semi-custom implants reduce the size of inventory and molds required for production, while allowing some degree of patient-specific customization.

Custom or patient-specific implants, when approved by surgeon at 170 for a specific patient, can be manufactured for the patient by rapid prototyping methods, such as stereolithography or other similar methods, or by CNC milling, or other automated or computer-controlled machining, or by robotic methods, at 250. Manufacturing can take place at a manufacturing center or facility in situ or at remote or off-site location. It will be understood that in situ manufacturing is used as a short hand for a manufacturing site of the original equipment manufacturer (OEM), but can be physically located at a different facility of the OEM. Off-site or remote manufacturing will be understood to refer to facilities operated by other manufacturers who are contracted by the OEM for manufacturing all or some of the components or parts for the surgical procedure.

Off-the-shelf implants, when approved by the surgeon a 190, can be manufactured by standard casting methods from bar stock or other stock material at 200, then shaped to a final shape and size by grinding or milling at 210, polished at 220, and then cleaned/passivated at 230. Such off-the-shelf implants can be part of an existing inventory, or mass-produced, or produced by just-in-time agile manufacturing methods.

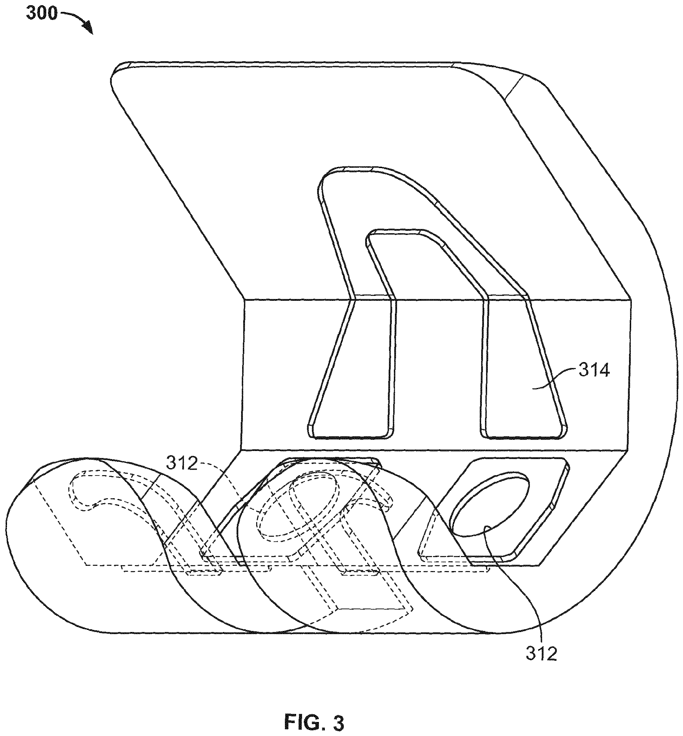

Semi-custom implants, when approved by the surgeon at 180, can be made from a generic casting at 240, as described below, or by modifying existing standard implant designs to match various features or parameters based on the anatomy of the patient, as described in co-pending patent application entitled Patient-Modified Implant and Associated Method, Serial No. 12/103834, filed on April 16, 2008, the disclosure of which is incorporated by reference herein. After the generic casting is modified for certain parameters of a patient, it can be processed at aspects 210-230 to a passivated form. Patient-specific parameters can include parameters relating to the size of the implant, including height, width, various articulation parameters or angles, etc., as discussed in specific example below in reference to FIGS. 3-5.

The surgeon's review of the surgical plan at 140 may further include, at 160, a request for one or more patient-specific alignment guides to be used with the implant. Patient-specific alignment guides are described in co-pending patent applications Serial No. 11/756,057, filed on May 31, 2007, Serial No. 11/971,390, filed on January 9, 2008, No. 12/025,414, filed on February 4, 2008, and Serial No. 12/039,849 filed on February 29, 2008. The alignment guides can be manufactured at 260 with by rapid prototyping methods, such as stereolithography or other similar methods or by CNC milling, or other automated or computer-controlled machining or robotic methods, and cleaned at 270. The alignment guides, the implants and optionally other disposable instruments can be packaged and sterilized at 280, and forwarded to the surgeon or the surgeon's medical facility for implantation at 290.

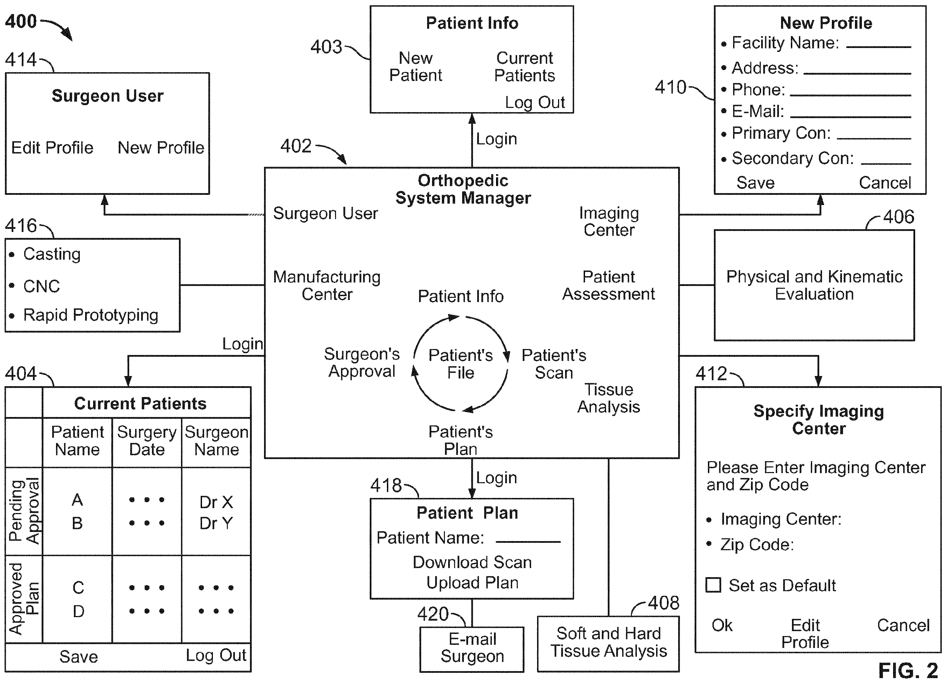

Referring to FIG. 2, a computer interface 400 to a computer program for the management of the manufacturing method is illustrated diagrammatically. An orthopedic system manager 402 can be in the form of software or other computer program associated with the original equipment manufacturer. The orthopedic system manager 402 can be accessible locally via dedicated computer machines or computer terminal directly communicated with software either by hard wire or wirelessly. The orthopedic system manager 402 can also be accessible remote remotely via the Internet or other remote communication portals using any electronic or other devices that can connect to the Internet or other web-based network, or other similar communication networks, including cable, satellite and telephone-based networks.

The system manager 402 can provide access to patient file information, including lists of all current patients at 403, and surgery dates, surgeons, and approval status of the surgical plan for each patient, at 404. Each patient file can include personal and medical information of the patient, such as, for example, weight, height, gender, age, lifestyle, pertinent medical records and medical history, as well as information on patient assessment that includes physical and kinematic evaluation pertaining to the orthopedic procedure at 406, and soft and hard tissue analysis at 408, including information provided at aspects 120 and 125 of FIG. 1, as discussed above. Imaging center information for patient scans, as discussed in relation to aspects 100 and 110 of FIG. 1, can added or modified at 410, and an imaging center for each specific patient can be specified at 412. Surgeon profiles, including surgeon preferences regarding anatomic axes alignment or implant and instrument preferences that can be taken into account when preparing the preliminary pre-operative plan discussed at aspect 130 of FIG. 1, can be created and edited at 414. Information and selection of manufacturing centers can be accessed at 416 for manufacturing the implants and or alignment guides as discussed in relation to aspects 260, 250, 240, and 210-230 of FIG. 1. The preliminary pre-operative surgical plan for each patient can be provided at 418, as discussed above at 140 in reference to FIG. 1, and e-mailed or otherwise communicated to the patient's surgeon at 420.

As discussed above at aspects 150 to 190 of FIG. 1, one implant option includes manufacturing semi-custom implants by generic casting. Illustrative examples of generic casting of a semi-custom femoral component are shown in FIGS. 3-5. A generic casting 300 of the implant is a casting that is more specialized than ordinary bar stock, from which any size of component can be made, but less specialized than the off-the-shelf components that are available in a particular number of sizes, typically six-to ten sizes and are finished from specific castings of those sizes. The generic casting can be made in a size and shape that can accommodate a range of variable features for the component, and at the same time can be machined to multiple sizes, such as three or four smaller sizes. In contrast, off-the-shelf implants require a mold or casting for each offered size, and a larger inventory of available sizes for each implant component. The generic casting can generally include geometric features which are size/shape and/or patient-independent or universal, and also features that are size/shape or patient-specific, as discussed in the examples below. More particularly, the generic casting can include at least one geometric feature that will remain unchanged for any patient or universal feature, and at least one geometric feature that can be specifically customized for and is specific to a particular patient.

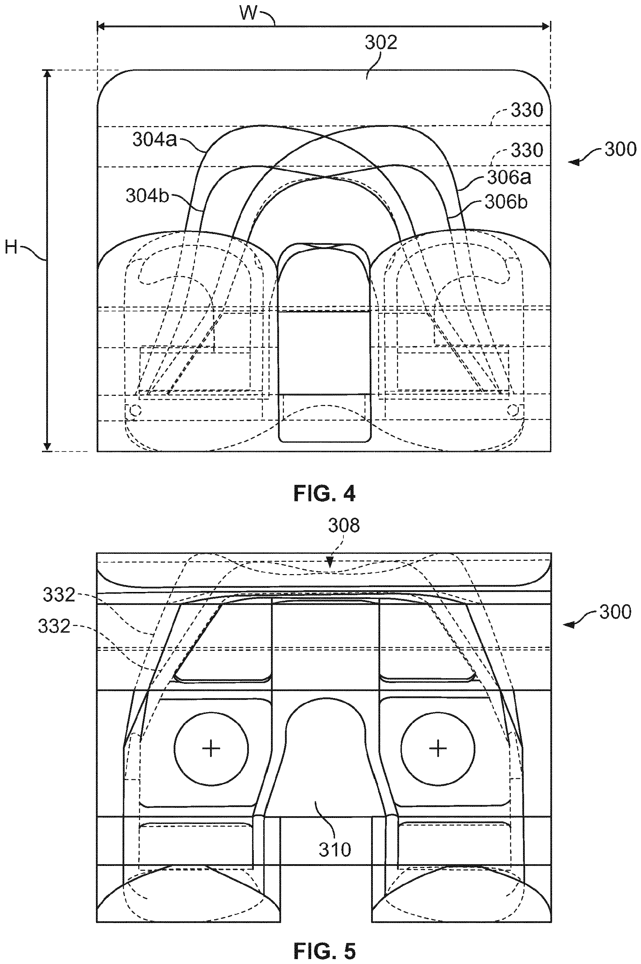

Referring to FIGS. 4 and 5, an exemplary generic casting 300 of a femoral component is illustrated. In this example, the generic casting 300 can have an anterior flange 302 of medial-lateral width W, and/or a height H and/or other geometric dimensions to accommodate multiple sizes of femoral components. For example, multiple sizes of left-sided implants 304a, 304b, and various sizes of right-sided implants 306a, 306b can be formed by a single generic casting. Appropriate markings or indentations or score lines for cutting to size can be provided, such as height markings 330, for example. The implant for a particular patient can be formed from the generic casting 300 by selecting particular features, such as the width W or height H, or other geometric features for a particular patient and machining the generic casting 300 to provide the size, dimension or shape, or combinations thereof for that particular geometric feature.

Referring to FIG. 5, the generic casting 300 does not include a patella track feature, but provides an area in which a custom patella track 308 can be machined at a custom angle for each specific patient. The generic casting 300 can also include additional material in the inner condylar notch area 310 to allow for custom machining of the intercondylar notch area 310 to accommodate various types of articulation or constraint in relation to a tibial component, such cams or intercondylar boxes, and other contact areas for articulation with the tibial component in accordance with a kinematic plan for the joint of the specific patient. Separate molds for posterior stabilized and cruciate retaining articulations can be made, each mold capable of accommodating multiple sizes of the corresponding implant type. For example, the intercondylar notch area 310 can be machined for line or area contact with the articular surfaces of a tibial component of various degrees of flexion. Exemplary articulations are disclosed in commonly assigned U.S. Patents No. 6,589,283, No. 6,413,279, and No. 6,165,223, and in co-pending U.S. Patent Application Ser. No. 10/840,765 filed on May 6, 2004, all of which are incorporated herein by reference. Various markings 332 corresponding to different sizes can be provided.

Referring to FIG. 3, the generic casting 300 can include at least one patient-independent or universal feature, such as, for example, universal cement wells 312 or other universal features. Such universal features can be used with any internal geometry 314, which can be machined into the generic casting 300 to accommodate the appropriate shape and/or size for a specific patient.

It will be appreciated from the above discussion that generic casting can greatly reduce inventory, machining costs and investment in mold tooling, while at the same time accommodating sizes and geometric features specific to a patient. Specifically, each implant type can be formed from a generic casting that can accommodate multiple sizes, such as four sizes, for example. For implants that are available in eight sizes, generic casting can reduce inventory by a half, using two molds total for eight sizes. Further, additional reductions in inventory can be obtained by combining right and left side implants into a single generic casting, as discussed above in relation to FIG. 4.

The process can then be followed as illustrated in Figs. 13A and 13B as discussed above in relation to Fig. 1. The blocks in Figs. 13A and 13B that are illustrated with the same reference numerals as in Fig. 1, but augmented with a prime are not discussed in further detail, but are discussed above in Fig. 1 and includes substantially similar processes. In addition to the various applications discussed above, input from a surgeon or other appropriate user can be provided with a handheld device, as discussed above. As illustrated in Figs. 13A and B, a handheld device can be used by the surgeon to review the pre-operative plan at 140'. It will be understood, however, that the process for providing a selected implant and tools can be similar to that discussed above, for example, with reference to Fig. 1

With reference to Fig. 14, a schematic diagram illustrating main or exemplary hardware components for the process illustrated in Fig. 13A and 13B is illustrated. The pre-operative plan or preliminary pre-operative plan from block 130 and 130' can be developed or produced by a service provider 700. The service provider 700 can own, operate, manage, or the like a main processor 702 and a main memory 704. The main processor and main memory 702, 704 can be at the service provider 700, in communication with the service provider 700, or otherwise controlled, maintained, or used by the service provider 700. Further, the main processor and main memory 702, 704 can be incorporated into a single server system. Regardless, the main processor 702 can process or execute a program, such as a program to develop the pre-operative plan, accept inputs from the surgeon, and augment or generate the final pre-operative plan. In addition, the main processor 702 can also be used to design and output the implant and alignment guide in blocks 150, 150', and 160, 160'.

As discussed above, and further herein, the pre-operative plan can be delivered or accessed by the surgeon via notification or surgeon access in block 600', 602'. The access or delivery of the pre-op plan can be via an internet or worldwide web connection 706 that uses a first communication method 708 from the service provider 700 and a second communication method 710 to a handheld device 712. It will be understood that the first and second communication method 708, 710 can be wired or wireless and can both be the same. Alternatively, or in addition to an internet connection a cell or mobile phone connection system 714, such as a tower, cell phone, antenna, can be provided. A first communication line 716 can transmit a communication through the mobile phone connection system 714. It will be understood that the service provider can communicate directly with the mobile phone connection system 714 via connection 716 or though an indirect connection 716', such as an internet connection. A second communication line 718 can be used by a surgeon 720 with the handheld device 712. Again, it will be understood, that the first and second communication system 716, 716', and 718 can be wired or wireless and can be the same or different. In addition, an intermediate system, such as a laptop or desktop computer 722 can be in communication with a system, such as the internet 706 via a first communication system 724 and the handheld device 712 can be interconnected with the computer 722 via a second communication system 726. Again, the first and second communication system 724, 726 can be the same or different and be wired or wireless.

With further reference to Figs. 13A, 13B, and 14, the pre-operative plan from block 130' can be delivered to a surgeon or accessed by a surgeon in any appropriate manner, such as via the internet 706 or cell communication 714. The pre-operative plan can be the preliminary pre-operative plan as discussed above. The pre-operative plan can include or be saved as a data file, in the main memory 704 associated with the main processor 702 of the service provider 700, of an appropriate type including image data, patient data, resection area data, etc. The pre-operative plan can be generated and stored by the service provider 700. The service provider 700 can be any appropriate service, such as an implant and/or guide manufacturer or specification producer. A specification producer can be a a service that provides specifications for an implant or guide to a manufacturer for production.

The service provider 700 can notify the surgeon 720 or user that the preliminary pre-operative plan is ready for review in block 600'. The notification that the pre-operative plan is prepared can be performed in any appropriate manner. For example, an electronic mail notification can be sent to the surgeon 720, a text message can be sent to the surgeon 720, a telephone call can be made to the surgeon 720 via landline or a wireless connection, as illustrated in Fig. 14. Regardless, the surgeon can be notified that the pre-operative plan is ready for review in block 600 through the use of the mobile device 712.

Once the surgeon 720 is notified that the pre-operative plan is ready for review, the surgeon 720 can access the pre-operative plan in block 602'. The surgeon can access the pre-operative plan in one or a plurality of ways in block 140'. For example, the surgeon 720 can download the pre-operative plan to the handheld device in block 604'. Alternatively, or in addition thereto, the surgeon 720 can access the main processor/memory 702, 704 to review the pre-operative plan in the main memory 704 in block 606' with the handheld device 712. It will be further understood that the surgeon 720 may also access the plan with the computer or terminal 722 by downloading the pre-operative plan data file to the computer 722 on which appropriate software is installed to access the pre-operative plan. The surgeon 720 may also view a printout of the pre-operative plan for manipulating or commenting on the pre-operative plan, or any other appropriate manner.

If the surgeon 720 downloads the file to the handheld device 712, the file can be downloaded to the handheld device 712 using any appropriate transfer protocol or communication system, as illustrated in Fig. 14. For example, the handheld device 712 can be connected to the computer 722 through an appropriate communications cable or protocol 726, such as Bluetooth®, a wireless communication protocol or a Universal Serial Bus (USB) cable. Once the file is downloaded to the handheld device 712, a program on the handheld device 712 can execute or read the file and display images for the the surgeon 720. The surgeon 720 can then review the plan in block 140'. For example, as illustrated in Fig. 7, a view of a bone to be resected can be displayed along with the slider bars 520 for allowing editing or augmentation of the pre-operative plan by the surgeon.

The view of the images, including the slider bars 520, can be augmented for the handheld device 712. For example, a cropped and zoomed image may only include a portion of the bone or region to be cut or resected for viewing by the surgeon. For example, as illustrated in Fig. 15, the handheld device 712 can include a view screen 740 that displays an image of the bone to be resected, but only includes a portion thereof, such as a lateral portion of a distal femur. The zoomed and cropped image can include fewer than all of the slide bars 520, such as only the slide bars 520'c and 520'd. The slide bars 520'c and 520'd can be illustrated and accessed by the surgeon to change data in the file for creation of appropriate instruments and implants for the specific patient or for augmentation of semi-custom or selection of an off-the-shelf instrument and implant.

The surgeon 720 can access or change the pre-operative plan file using the slide bars 520'c and 520'd through any appropriate access or manipulation process. For example, the screen 740 of the handheld device 712 can be a touch screen. Accordingly, the surgeon 720 can touch the screen with a finger or stylus to move the slider or marker portion 525 on the slider bars 520'c and 520'd. Alternatively, or in addition thereto, a trackball or other pointer device 742 can be provided to access and move the slider bars 520'c and 520'd. It will be understood that the handheld device 712 can have any appropriate input devices, such as an external or connected input devices, that can be mapped for appropriate command inputs into the system for augmenting the pre-operative plan file. The changes, if made by the surgeon, can then be saved to the pre-operative plan file to generate an edited pre-operative plan file.

If the surgeon 720, after review of the pre-operative plan in block 140', finds the plan to be unacceptable in block 620', the NO path 622' can be followed for the surgeon 720 to edit the plan in block 624'. The surgeon 720 can edit the plan in block 624 in any appropriate manner, such as by moving the slider bars 520'c and 520'd, or any other appropriate slider bars. Alternatively, the surgeon 720 can edit the pre-operative plan in any appropriate manner, including those discussed above. Moreover, the surgeon 720 can input changes into the pre-operative plan using any appropriate input portion, such as touching the screen 740. It will be understood, that the pre-operative plan file that is downloaded to the handheld device 712 can then be saved with the edits as the edited pre-operative plan and transmitted to the service provider 700 for appropriate edits to be re-reviewed or finalized, as discussed above. It will be understood, however, that the handheld device 712, which can include the appropriate program, can save the file in the appropriate format and transmit it back to the service provider 700.

Alternatively, or in addition to downloading the pre-operative plan file, the handheld device 712 can access the pre-operative plan which is stored in the main storage 704 associated with the service provider in block 606 and separate from the handheld device 712. If the handheld device 712 accesses the pre-operative plan on the main processor, memory 702, 704, the handheld device 712 need only display an image representing a portion of the file on the display screen 740. That is, the pre-operative plan and any edits or processing made to the pre-operative plan can be done solely or substantially by the main processor 702 that executes a program to manipulate and display the file. The main processor 702 and the main memory 704 need not be physically near or connected to the handheld device 712.

The handheld device 712 can be provided to display the image, such as an image of the bone for resection, for the surgeon 720. Therefore, the handheld device 712 may not be required to process the pre-operative plan file from the service provider 700, but only be provided to display the pre-operative plan file and receive and transmit input from the surgeon 720. Accordingly, even if the pre-operative plan is accessed from the service provider in block 606, the slide bars 520'c and 520'd can be displayed on the display 742 of the handheld device 712 for input by the surgeon 720. The inputs, however, can be directly transmitted to the main processor 702 for processing augmentation or editing of the file. This is in addition or alternative to augmenting or editing the file that has been downloaded to the handheld device 712 for re-transmission of the edited pre-operative plan to the service provider 700.

By only or substantially accessing the pre-operative plan file from the main processor/ memory 702, 704 data transmission can be minimized from the main memory 704 or provider 700 to the handheld device 712 of the surgeon 720. Decreased data transmission can provide increased speed and decreased data usage costs or bottlenecks in a system. In addition, the handheld device 712 can be provided or include limited memory and processing capabilities when the pre-operative plan file is only accessed with the handheld 712 and only small amounts of information are transferred, for example, regarding slide bar location and smaller portions of an image file. Accordingly, it can be provided, that a complete or pre-operative plan is transmitted to the handheld device 712, processed completely on the handheld device 712, edited on the handheld device 712, saved and re-transmitted back to the service provider 700 or the handheld device 712 can only access the pre-operative plan file saved at the main memory 702 and transmit edits to the server.

Data transmission and processing can also be reduced by limiting or cropping the pre-operative plan data file. For example, as illustrated in Fig. 15, only a lateral and distal portion of the bone is illustrated on the display device or display screen 740 of the handheld device 712. It will be understood that an image file or image information can include the entire bone or other data saved in the image file. Accordingly, the image file can be cropped at the server or at the provider and only a portion of the image file transmitted to the handheld device 712. This can be done repeatedly for different portions of the image data to allow for smaller file packet size or file size for transmission to the handheld device 712. The cropping and compression of the data file can be done in substantially real-time by the server for a substantially seamless viewing and manipulation by the surgeon.

Even if the surgeon 720 accesses the data file on the main memory 704, the surgeon 720 can review the pre-operative plan block 140', as discussed above, and make a determination of whether the pre-operative plan is acceptable in block 620'. As discussed above, if the pre-operative plan is not acceptable, the NO path 622' can be followed to allow for surgeon edits in block 624'.

Further, regardless of the method of review of the pre-operative plan in block 140', the pre-operative plan can be determined to be acceptable in block 620' and follow the YES path 626'. When following the YES path 626', the implant can be designed and an alignment guide can be designed in blocks 150' and 160'.

The foregoing discussion discloses and describes merely exemplary arrangements of the present teachings. Furthermore, the mixing and matching of features, elements and/or functions between various embodiments is expressly contemplated herein, so that one of ordinary skill in the art would appreciate from this disclosure that features, elements and/or functions of one embodiment may be incorporated into another embodiment as appropriate, unless described otherwise above. Moreover, many modifications may be made to adapt a particular situation or material to the teachings of the invention without departing from the essential scope thereof. One skilled in the art will readily recognize from such discussion, and from the accompanying drawings and claims, that various changes, modifications and variations can be made therein without departing from the spirit and scope of the present teachings as defined in the following claims.

## Claims

1. A method of preparing a pre-operative plan for an operative procedure regarding a patient, comprising:
operating a handheld device to augment a preliminary preoperative plan;
viewing the pre-operative plan with a display as the handheld device;
editing the preliminary pre-operative plan through the handheld device; and
saving an edited pre-operative plan based on editing the preliminary pre-operative plan.

2. The method of Claim 1, further comprising:
acquiring image data of the patient;
determining a healthy anatomical orientation of the patient;
selecting resections and implants to achieve the determined substantially anatomical orientation after performing the operative procedure based on the pre-operative plan; and
generating the preliminary pre-operative plan at a provider based on at least one of the determined substantially anatomical orientation of the patient and selected resections and implants.

3. The method of Claim 2, wherein accessing the preliminary pre- operative plan includes: downloading the preliminary pre-operative plan to a handheld device.

4. The method of Claim 3, wherein downloading the preliminary pre- operative file includes transmitting the data file wirelessly via a cellular or mobile phone data network or wherein downloading includes transmitting wirelessly to the handheld device via a local access wireless transmission data protocol or wherein downloading includes transmitting to the handheld device via a cable connection or physical connection to a main processor and main storage system.

5. The method of Claim 3, wherein viewing the pre-operative plan includes viewing a cropped portion of an image of the pre-operative plan and optionally further comprises: zooming into the cropped portion of the image of the pre-operative plan to view in detail a portion of the cropped portion of the pre-operative plan and optionally further comprises editing only the cropped portion of the image of the pre-operative plan.

6. The method of Claim 3, further comprising editing the preliminary pre-operative plan including executing a program on the handheld device to edit the preliminary pre-operative plan on the handheld device and optionally wherein saving the edited pre-operative plan includes saving the edited pre-operative plan in a storage system of the handheld device.

7. The method of Claim 1, wherein accessing a preliminary preoperative plan includes accessing the preliminary pre-operative plan stored on a main storage system associated with a main processor separate from a handheld device; wherein accessing the preliminary pre-operative plan includes wirelessly accessing the preliminary pre-operative plan with a selected wireless communication system and protocol.

8. The method of Claim 7, wherein the selected wireless data system and protocol includes a cellular or mobile wireless data system.

9. The method of Claim 7, further comprising: transmitting to the main processor via the wireless transmission system, the provided inputs from the handheld device; wherein editing the preliminary pre-operative plan includes executing a program with the main processor to edit the preliminary preoperative plan to generate the edited pre-operative plan.

10. The method of Claim 9, wherein saving the edited pre-operative plan includes saving the edited pre-operative plan at the main storage system associated with the main processor separate from the handheld device and optionally wherein editing the preliminary pre- operative plan and saving the edited pre-operative plan occurs substantially separate from the handheld device.

11. The method of claim 1, wherein the preliminary pre-operative plan includes:
a three-dimensional image of the patient's un-resected distal femoral bone created from MRI, CT, ultrasound, radiography or X-ray images of the patient's knee joint; and
a planned bone resection plane or planes.

12. The method of claim 11, wherein viewing the pre-operative plan with a display on the handheld device comprises viewing on the display of the handheld device the resection planes shown in the three dimensional image of the patient's knee joint.

13. The method of claim 1, wherein editing the preliminary pre-operative plan through the handheld device comprises:
modifying the image of the femoral bone viewed on the display of the handheld device by making changes in the location, size or orientation of the resection planes and/or removing one or more osteophytes/protrusions from the image or
providing inputs with the handheld device based on the viewed image.

14. The method of claim 13, further comprising selecting on the display, a femoral knee implant corresponding to the modified image of the femoral bone

15. The method of claim 14, further comprising:
transmitting the edited pre-operative plan to an implant manufacturer; and
requesting manufacture of the femoral knee implant according to the edited pre-operative plan and the modified image of the distal femoral bone.

16. An orthopedic implant manufacturing method comprising: preparing a pre-operative surgical plan for a specific patient, the surgical plan including a three-dimensional image of a patient's joint indicating at least one resection plane; communicating the surgical plan to a surgeon of the patient; receiving approval of the surgical plan and the resection plane by the surgeon; providing automated osteophyte/protrusion removal control for surgeon manipulation; receiving a modified three-dimensional image of a patient's joint indicating an osteophyte/protrusion removal and a recommendation for a corresponding selected orthopedic implant from the surgeon; and requesting manufacture of the selected orthopedic implant.

17. The method of claim 16, wherein providing automated osteophyte/protrusion removal control for surgeon manipulation includes identifying a location of at least one osteophyte/protrusion on the three- dimensional image of a patient's joint.

18. The method of claim 17, wherein providing automated osteophyte/protrusion removal control for surgeon manipulation includes providing a plurality of depth contours in relation to the osteophyte/protrusion.

19. The method of claim 18, wherein providing automated osteophyte/protrusion removal control for surgeon manipulation includes providing at least one graphical removal tool associated with the osteophyte/protrusion for manipulation by the surgeon.

20. The method of claim 18, wherein the plurality of depth contours is continuous.

21. The method of claim 20, further comprising referencing the plurality of depth contours from a landmark location associated with the osteophyte/protrusion.

22. The method of claim 17, wherein identifying a location of an osteophyte/protrusion on three-dimensional image of a patient's joint includes determining a tissue overhang protruding from an edge of a resection plane.

23. The method of claim 16, further comprising receiving a recommendation for a selected alignment guide from the surgeon.

24. The method of claim 23, further comprising providing the selected alignment guide and selected orthopedic implant to the surgeon in one package.

25. The method of claim 16, wherein the selected orthopedic implant is a femoral joint component.

26. The method of claim 25, wherein the femoral joint component is custom made for a specific patient.

27. The method of claim 25, wherein the femoral joint component is semi-custom made.

28. The method of claim 16, wherein the selected orthopedic implant is a tibial joint component.

29. The method of claim 28, wherein the tibial joint component is custom made for a specific patient.

30. The method of claim 28, wherein the tibial joint component is semi- custom made.

31. An orthopedic implant manufacturing method comprising: preparing a pre-operative surgical plan for a specific patient, the surgical plan including a three-dimensional image of a patient's joint indicating at least one resection plane; communicating the surgical plan to a surgeon of the patient; receiving approval of the surgical plan and the resection plane by the surgeon; identifying a location of at least one osteophyte/protrusion on the three-dimensional image of a patient's joint; providing a plurality of depth contours in relation to the osteophyte/protrusion ; providing at least one graphical removal tool associated with the osteophyte/protrusion for manipulation by the surgeon; receiving a modified three-dimensional image of a patient's joint indicating an osteophyte/protrusion removal and a recommendation for a corresponding selected orthopedic implant from the surgeon; and requesting manufacture of the selected orthopedic implant.

32. The method of claim 31, wherein the plurality of depth contours is continuous.

33. The method of claim 32, further comprising referencing the plurality of depth contours from a landmark location associated with the osteophyte/protrusion.

34. The method of claim 32, wherein identifying a location of an osteophyte/protrusion on three-dimensional image of a patient's joint includes determining a tissue overhang protruding from an edge of a resection plane.

35. The method of claim 32, further comprising receiving a recommendation for a selected alignment guide from the surgeon.

36. An orthopedic implant manufacturing method comprising: preparing a pre-operative surgical plan for a specific patient, the surgical plan including a three-dimensional image of a patient's joint indicating at least one resection plane; identifying a location of at least one osteophyte/protrusion on the three-dimensional image of a patient's joint; providing a plurality of depth contours in relation to the osteophyte/protrusion ; providing at least one graphical removal tool associated with the osteophyte/protrusion for manipulation by a user; communicating the surgical plan to a user; receiving a modified three-dimensional image of a patient's joint indicating an osteophyte/protrusion removal and a recommendation for a corresponding selected orthopedic implant from a user; and requesting manufacture of the selected orthopedic implant.

37. The method of claim 36, further comprising: receiving the selected orthopedic implant; and forwarding the selected orthopedic implant for implantation.

38. A method of preparing a pre-operative plan for an operative procedure regarding a patient, comprising: accessing a data file including a preliminary pre-operative plan with a handheld device; viewing the pre-operative plan with a display on the handheld device; providing inputs with the handheld device; editing the preliminary pre-operative plan based on the provided inputs; and saving an edited pre-operative plan.

39. The method of Claim 38, further comprising: generating the preliminary pre-operative plan at a service provider; and transmitting the edited pre-operative plan to the service provider.

40. The method of Claim 38, further comprising: requesting manufacture of a selected orthopedic implant.

41. The method of Claim 40, further comprising: designing a selected orthopedic implant based on the transmitted edited pre-operative plan.

42. The method of Claim 41, further comprising: receiving the selected orthopedic implant; and forwarding the selected orthopedic implant for implantation.

43. The method of Claim 42, further comprising: acquiring image data of the patient; determining a healthy anatomical orientation of the patient; and selecting resections and implants to achieve the determined substantially anatomical orientation after performing the operative procedure based on the pre-operative plan.

44. The method of Claim 42, wherein generating the preliminary preoperative plan includes at least one of determining a substantially anatomical orientation of the patient and selecting resections and implants to achieve the determined substantially anatomical orientation after performing the operative procedure based on the pre-operative plan.

45. The method of Claim 38, wherein accessing a data file includes copying the data file to the handheld device and executing a program to process the data file for viewing with the handheld device.

46. The method of Claim 45 wherein editing the preliminary preoperative plan includes: executing a program with the handheld device based on the provided inputs.

47. The method of Claim 38, wherein accessing a data file includes: communicating with a main processor to transmit information regarding an image to the handheld device; and viewing the image on the handheld device.

48. The method of Claim 47, wherein editing the preliminary preoperative plan includes: transmitting the provided inputs with the handheld device to the main processor; and executing a program only with the main processor to change the preliminary pre-operative plan to the edited pre-operative plan; wherein saving an edited pre-operative plan includes saving the edited pre-operative plan at a location separate from the handheld device.

49. A method of preparing a pre-operative plan for an operative procedure regarding a patient, comprising: generating a preliminary pre-operative plan data file at a service provider based on at least one of a determined substantially anatomical orientation of the patient and selected resections and implants to achieve the determined substantially anatomical orientation of the patient; notifying a user that the preliminary pre-operative plan has been generated; formatting at least a portion of the preliminary pre-operative plan for display on a handheld device; delivering the formatted portion of the preliminary pre-operative plan; viewing the formatted portion of the pre-operative plan with a display on the handheld device; editing the preliminary pre-operative plan data file via inputs transmitted from the handheld device; and saving an edited pre-operative plan.

50. The method of Claim 49, further comprising: viewing an image formatted for viewing on the display device from the formatted portion of the pre-operative plan; and providing inputs with the handheld device based on the viewed image; wherein editing the preliminary pre-operative plan data file includes changing at least one parameter of the preliminary pre-operative plan data file based on the provided inputs.

51. The method of Claim 50, wherein providing inputs with the handheld device includes touching the display device to move a slider illustrated on the display device.

52. The method of Claim 49, further comprising: acquiring image data of the patient; determining a substantially anatomical orientation of the patient; and selecting resections and implants to achieve the determined substantially anatomical orientation after performing the operative procedure based on the pre-operative plan.
